# EUROPEAN PATENT SPECIFICATION

(11) **EP 3 541 200 B1**
(45) Date of publication and mention of the grant of the patent: **03.09.2025**
(21) Application number: 17797665.1
(22) Date of filing: 17.11.2017
(51) Int. Cl.: A23K 20/105, A61K 9/14, A23K 20/111, A23K 20/121, A23K 50/10, A61K 31/04, A61K 31/24, A61K 31/345

(54) **POWDEROUS FORMULATIONS**
PULVERFÖRMIGE FORMULIERUNGEN
FORMULATIONS PULVÉRULENTES

(30) Priority: 18.11.2016 EP 16199547
(43) Date of publication of application: 25.09.2019
(73) Proprietor: DSM IP Assets B.V., 6221 BE Maastricht (NL)
(72) Inventor: GADIENT, Martin, Reto, 4303 Kaiseraugst (CH); VIDONI, Olivia, Brigitte, 4303 Kaiseraugst (CH)
(74) Representative: dsm-firmenich IP
(86) International application number: PCT/EP2017/079567
(87) International publication number: WO 2018/091643

(56) References cited:
- EP-A1- 1 132 009
- EP-A1- 1 790 234
- WO-A1-2010/079104
- WO-A1-2011/070133
- WO-A1-2012/084629
- WO-A1-2013/156574

## Description

The present invention relates to improved formulations of 3-nitrooxypropanol as well as to the production of such formulations.

The temperature of the air surrounding the earth is increasing, a process referred to as global warming. One of the main focuses to reduce this warming effect is to reduce the amount of greenhouse gases emitted into the atmosphere. Greenhouse gases are emitted from several different sources, both natural and artificial; however the two sources with the most emphasis are the agricultural and fossil fuel industries. Within agriculture, ruminants and in particular cattle are the major contributors to the biogenic methane formation, and it has been estimated that the prevention of methane formation from ruminants would almost stabilize atmospheric methane concentrations.

3-Nitrooxy propanol and structural analogues thereof have been reported to be highly efficient in reducing the formation of methane in ruminants without affecting microbial fermentation in a way that would be detrimental to the host animal (WO2012/084629).

However, 3-nitrooxypropanol and structural analogues thereof are volatile and have been found not to be effectively retained in standard carrier systems commonly used in the feed industry under conventional storage conditions, which in turn may lead to a significant loss of the active in the final product form such as the feed product or the requirement of elaborate (i.e. tight and expensive) packaging systems.

Thus, there is an ongoing need to develop a product form, which overcomes the above-mentioned problem, i.e. a product form which avoids the evaporation of 3-nitrooxypropanol during storage, and additional exhibits a good flowability and which can easily be admixed with other components commonly used in feed products for ruminants.

Surprisingly, it has been found that 3-nitrooxypropanol is effectively retained in sepiolite as carrier under conventional storage conditions.

WO2013156574 discloses sepiolite as a carrier for (other) CH4-reducing feed components.

Illustrative to the present claimed invention are disclosed powderous formulation (I) comprising
(i) At least 0.1 weight-% (wt-%), based on the total weight of the powderous formulation, of a compound of formula (I) 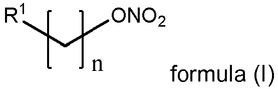 wherein
   n is an integer from 1 to 46
   R¹ is H, C₁-C₆alkyl, phenyl, -OH, -NH₂, -CN, -COOH, -O(C=O)R⁸, -NHC(=O)R⁸, SO₂NHR⁸, or -ONO₂, and
   R⁸ is C₁-C₆alkyl, phenyl, pyridyl such as preferably 2-pyridyl
   with the proviso that when z is > 3 the hydrocarbon chain may be interrupted by -O- or -NH-.
(ii) 0 wt-% - 35 wt-%, based on the total weight of the powderous formulation, of an edible solvent, and
(iii) at least 40 wt-%, based on the total weight of the powderous formulation, of sepiolite.

It is well understood that the above compositions are storage stable, i.e. exhibit a retention of at least 70 %, preferably of at least 80 % most preferably of at least 90 % of the compound of formula (I) as illustrated in the examples.

The formulations as disclosed above are powders, which depending on the process of production as well as the storage conditions, may comprise some water. The water content is usually below 5 wt-%, based on the total weight of the formulation. Therefore the formulations as described above, wherein 0 to 5 wt-%, based on the total weight of the formulation, of water is present, are described.

The formulations may furthermore contain small amounts of customary additives commonly used in the preparation of powderous formulations for feed application. Therefore, formulations, wherein 0 to 5 wt-%, based on the total weight of the formulation, of an additive is present, are described.

It is clear that the addition of all the wt-% always add up to 100. However, it cannot be excluded that small amount of impurities may be present such as e.g. in amounts of less than 5 wt.-%, preferably less than 3 wt.-% which are introduced via the respective raw materials or processes used.

The compound of the claimed invention of formula (I) is 3-nitrooxypropanol (CAS-No: 100502-66-7). Illustrative other compounds of formula (I) are 9-nitrooxynonanol, 5-nitrooxy pentanoic acid (CAS 74754-56-6), 6-nitrooxy hexanoic acid (CAS 74754-55-5), Bis(2-hydroxyethyl)amine dinitrate (CAS 20830-49-3), 1,4-bis-nitrooxybutane (CAS 3457-91-8) and 1,5-bis-nitrooxypentane (CAS 3457-92-9). 3-nitrooxypropanol and the illustrative compounds of formula (I) preferably have a boiling point below 250°C at 760 Torr, preferably a boiling point between 100 and 200°C at 760Torr.

In another embodiment, 3-nitrooxypropanol exhibits a vapor pressure in the range of 0.4 to 0.8 mbar, preferably in the range of 0.5 to 0.7 mbar, as then the problem of insufficient retention is particularly pronounced.

The compounds described herein are known and either commercially available or can be prepared in analogy to the processes as e.g. disclosed in WO2012/084629.

The term sepiolite as used herein refers to a soft white clay mineral consisting of hydrous magnesium silicate, a typical chemical formula for which is Mg₄Si₆O₁₅(OH)₂*6H₂O and which can be present in fibrous, fine-particulate, and solid forms. Commercially available sepiolite grades suitable for the purpose of the present invention encompass EXAL H 562, 1530 and 3060 which are commercially available from Tolsa (Spain).

Preferably the sepiolite according to the present invention has an average particle size D(v, 0.5) selected in the range of 100 to 1500µm, more preferably in the range of 200 to 1250 µm and most preferably in the range of 200 to 1250 µm.

In a more advantageous embodiment, the sepiolite according to the present invention has a D(v, 0.5) selected in the range of 200 to 1250 µm, and in particular also a D(v, 0.1) selected in the range of 100 to 800 µm and a D(v, 0.9) selected in the range of 300 to 1750 µm.

In a particular advantageous embodiment, the D(v, 0.5) is selected in the range of 800 to 1200 µm, preferably in the range of 900 to 1100 µm as this even further improves the retention of the active. In this case, it is further advantageous to select the D(v, 0.1) in the range of 500 to 800 µm and the D(v, 0.9) in the range of 1300 to 1750 µm.

The particle sizes as given herein are measured by a Malvern Master Sizer 2000 following the recommendations outlined in ISO13320-1 for particle size analysis via laser diffraction methods (laser diffraction light scattering). During this laser diffraction measurement, particles are passed through a focused laser beam. The particles scatter light at an angle that is inversely proportional to their size. The angular intensity of the scattered light is then measured by a series of photosensitive detectors. The map of scattering intensity versus angle is the primary source of information used to calculate the particle size. For the measurement of sepiolite, a dry powder feeder (Malvern Scirocco) was used.

The term edible oil refers to oils commonly used in feed applications. Preferred edible oils in all embodiments of the present invention are propyleneglycol, corn oil, rapeseed oil, sunflower oil, middle chain triglyceride (MCT) and glycerol as well as mixtures thereof. Most preferred in all embodiments of the present inventions is the use of propyleneglycol.

The term additive as used herein refers to additives commonly used in the preparation of powderous formulations for feed application such as in particular to thickeners which are advantageously selected from gums or cellulose derivatives such as xanthan gum, karaya gum and/ or ethylcellulose.

Preferred embodiments of the present invention are formulations (II), which comprise
(i) from 1 to 20 wt-%, based on the total weight of the powderous formulation, of 3-nitrooxypropanol, and
(ii) 5 wt-% to 35 wt-%, based on the total weight of the formulation, of at least one edible oil,
(iii) at least 50 wt-%, based on the total weight of the powderous formulation, of sepiolite,
(iv) 0 to 10 wt-%, based on the total weight of the powderous formulation, of water and/ or an additive.

A more preferred embodiment of the present invention relates to a formulation (III) consisting of
(i) from 2 to 15 wt-%, based on the total weight of the powderous formulation, of 3-nitrooxypropanol, and
(ii) 10 wt-% to 35 wt-%, based on the total weight of the powderous formulation, of an edible oil, and
(iii) at least 60 wt-%, based on the total weight of the powderous formulation, of sepiolite, and
(iv) 0 to 10 wt-%, based on the total weight of the powderous formulation, of water and/ or an additive.

An especially preferred embodiment of the present invention relates to a formulation (IV) consisting of
(i) 2 to 10 wt-%, based on the total weight of the powderous formulation, of a 3-nitrooxypropanol, and
(ii) 20 to 30 wt-%, based on the total weight of the powderous formulation, of an edible oil, and
(iii) at least 70 wt-%, based on the total weight of the powderous formulation, of sepiolite, and
(iv) 0 to 5 wt-%, based on the total weight of the powderous formulation, of water.

A very specific formulation of the present invention is a formulation (V) consisting of
(i) 4 to 8 wt-%, based on the total weight of the powderous formulation, of 3-nitrooxypropanol, and
(ii) 20 to 30 wt-%, based on the total weight of the powderous formulation, of propyleneglycol, and
(iii) at least 70 wt-%, based on the total weight of the powderous formulation, of sepiolite, and
(iv) 0 to 5 wt-%, based on the total weight of the powderous formulation, of water.

Generally, in order to produce a powder according to the present invention (formulations (I), (II), (III), (IV), (V)) 3-nitrooxypropanol is, optionally diluted in the edible oil and, if present, the additive, sprayed onto or admixed with sepiolite.

It is also possible that 3-nitrooxypropanol is, optionally in the presence of an edible oil and, if present, the additive, diluted in an organic solvent suitable for the preparation of food or feed products such as e.g. dichloromethane, sprayed onto or admixed with sepiolite followed by evaporation of the organic solvent.

The powderous formulation according to the present invention can additionally be coated with customary coatings in the art such as wax or fats. If present, such coating is generally applied in amounts of 5 to 50 wt.-% based on the total weight of the powderous form. Advantageously, the coating comprises at least one wax and/or at least one fat, which has a dropping point of from 30 to 85 °C.

The dropping point of a material as used herein refers to the temperature (in °C) when the material begins to melt under standardized conditions. Thus, the material is heated so long until it changes the state of matter from solid to liquid. The dropping point is the temperature when the first dropping is released from the material. The determination of the dropping point (Tropfpunkt) is carried out as described in the standard norm DIN ISO 2176.

Particularly suitable waxes to be used as coating in the context of the present invention include organic compounds such as long alkyl chains, natural waxes (plant, animal) which are typically esters of fatty acids and long chain alcohols as well as synthetic waxes, which are long-chain hydrocarbons lacking functional groups.

Particularly suitable fats to be used as coating in the context of the present invention include a wide group of compounds which are soluble in organic solvents and largely insoluble in water such as hydrogenated fats (or saturated fats) which are generally triesters of glycerol and fatty acids. Suitable fats can have natural or synthetic origin. It is possible to hydrogenate a (poly)unsaturated fat to obtain a hydrogenated (saturated) fat.

Preferred examples of waxes and fats to be used as coating according to the present invention are glycerine monostearate, carnauba wax, candelilla wax, sugarcane wax, palmitic acid, stearic acid hydrogenated cottonseed oil, hydrogenated palm oil and hydrogenated rapeseed oil as well as mixtures thereof.

All the above disclosed formulations (I), (II), (III), (IV), (V) can be used as such or incorporated in feed products or feed additives, in particular to enhance the retention of 3-nitrooxypropanol therein.

Additionally, all the above disclosed formulations (I), (II), (III), (IV) and (V) can be used as such in the production of feed products or feed additives, in particular to enhance the retention of 3-nitrooxypropanol therein.

In another embodiment, the invention relates to the use of sepiolite to enhance the retention of 3-nitrooxypropanol with all the preferences and definitions as given herein. Preferably, the retention is at least 70 %, preferably at least 80 % most preferably at least 90 %.

In another embodiment, the present invention relates to a method of reducing the evaporation and/ or volatility of 3-nitrooxypropanol respectively to a method of improving the retention of 3-nitrooxypropanol, aid method comprising admixing 3-nitrooxypropanol with all the definitions and preferences as given herein with sepiolite. In a preferred embodiment, the amount of sepiolite is at least 40 wt-%. In an even more preferred embodiment, the method comprises the preparation of a formulation (I), (II), (III), (IV) or (V) as defined herein, as these formulations are particular suitable to effectively retain the active over storage.

In another embodiment, the present invention relates to method of reducing the evaporation and/ or volatility respectively to method of improving the retention of 3-nitrooxypropanol, said method comprising the step of preparing a powderous formulation (I), (II), (III), (IV) or (V). Preferably, the powderous formulation exhibits a retention of at least 70 %, preferably of at least 80 %, most preferably of at least 90 %.

The term 'retention' as used therein refers to a retention of 3-nitrooxypropanol with all the definitions and preferences as given herein over a storage time of 12 weeks (open bag; 25°C; 50 % relative humidity (r.H.)).

In a further advantageous embodiment, the invention relates to a method to enhance the retention of a compound of 3-nitrooxypropanol with all the definitions and preferences as given here-

in in a feed product, said method comprising the step of adding 3-nitrooxypropanol in the form of a formulation (I), (II), (III), (IV) or (V) to the feed composition.

In a further embodiment, the invention relates to a method to enhance the storage stability of a feed product comprising 3-nitrooxypropanol, said method comprising the step of adding 3-nitrooxypropanol in the form of a formulation (I), (II), (III), (IV) or (V) to the feed composition.

Preferably, the amount of the formulation (I), (II), (III), (IV) or (V) in the feed product is selected such, that the amount of 3-nitrooxypropanol is in the range of 0.01 to 50g/ kg of feed product, preferably in the range of 0.02 to 25g/ kg of feed product, most preferably in the range of 1 to 10g/ kg of feed product.

The term feed product refers in particular to ruminant feed compositions as well as to feed additives.

The invention is illustrated by the following Examples. All temperatures are given in °C and all parts and percentages are related to the weight.

### Examples

### Example 1

*Particle size determination:* The methodology described below followed the recommendations outlined in ISO13320-1 for diffraction light scattering techniques: the particle sizes of various sepiolite grades have been measured by a Malvern Master Sizer 2000 following the recommendation of ISO13320-1. An aliquot of about 5 grams of the material tempered at 25°C- 35 to 55% r.H is sampled into the vibrator hopper of the (Sirocco) dry dispersion unit. The flow aperture of the dispenser gate is set up on the way that the product flows for 30 seconds through the measurement zone using a tygon tube, at a vibration feed rate of 50%. A sample measurement at 0.5 bar of disperser pressure is taken for 30 seconds and a snap of 30000 (on the Malvern Mastersizer 2000). The sample pass through the focused beams of light (Helium-neon laser for the red light and solid-state light source for the blue) and scatter the light allowing a measurement of particles between 0.02 and 2000 micrometers. The particle diameters in volume (as outlined in table 1), are determined using Fraunhofer approximation.

*Preparation of the formulations:* To 80g of different type of sepiolite grades as outlined in table 1) as well as silica (silicic acid) and diatomaceous earth placed in a beaker 20g of a 20 wt.-% 3-nitrooxypropanol solution in propyleneglycol is added under gentle agitation at room temperature. After 5 minutes agitation, the adsorption is complete and a free-flowing powder is obtained.

*Stability study:* Two aluminium bags containing 5g of the respective formulation are stored open at 25°C under controlled atmosphere (50 % r.H). The concentration of 3-nitrooxypropanol was determined by HPLC using an Agilent High Performance Liquid Chromatography 1260 Infinity system, using an Aquasil C18, 150 x 3mm, 3µm column and detecting at 210 nm. The column oven was set to 23°C, the autosampler not temperature controlled. The mobile phase consisted of mobile phase A (940mL Milli-Q-water + 60ml acetonitrile + 1mL methane sulfonic acid) and mobile phase B (800ml Milli-Q-water + 200ml acetonitrile + 1mL methane sulfonic acid) which were used in gradient mode (0 min: 0 % B, 15 min: 0 % B, 15.5 min: 100 % B, 21 min: 100 % B, 21.5 min: 0 % B, 25 min: 0 % B (= end of run)) with a flow of 0.4 ml/min. The results (as relative concentration to the initial value (= 100%)) are presented Table 2.

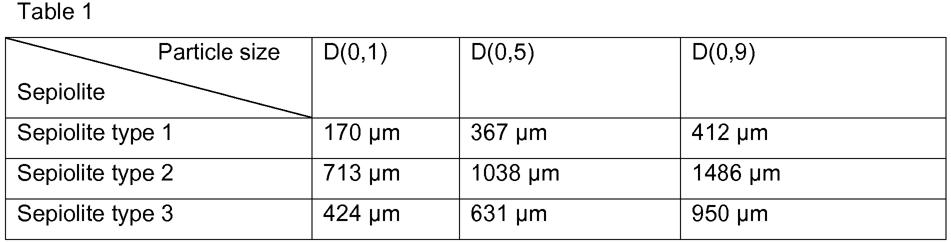

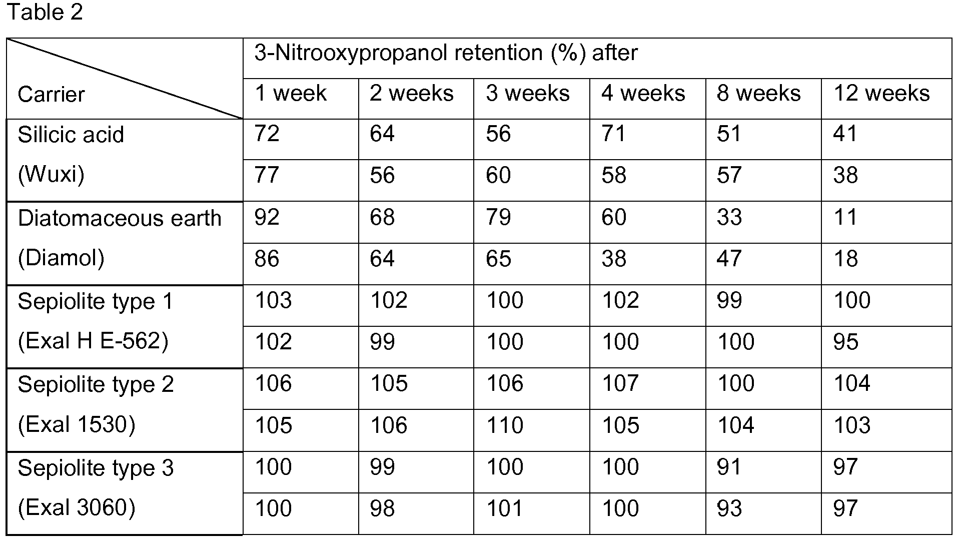

As can be retrieved from table 2, the use of a sepiolite carrier results in a significantly improved retention (i.e. significantly reduced evaporation) of the active compared to a silica respectively diatomaceous earth carrier.

## Claims

1. A storage-stable powderous formulation comprising
(i) At least 0.1 weight-% (wt-%), based on the total weight of the powderous formulation, of 3-nitrooxypropanol,
(ii) 0 wt-% - 35 wt-%, based on the total weight of the powderous formulation, of an edible solvent, and
(iii) at least 40 wt-%, based on the total weight of the powderous formulation, of sepiolite,

2. The powderous formulation according to claim 1 further comprising
(iv) 0 to 10 wt-%, based on the total weight of the powderous formulation, of water and/ or an additive.

3. The powderous formulation according to any of the preceding claims consisting of
(i) 2 to 15 wt-%, based on the total weight of the powderous formulation, of 3-nitrooxypropanol, and
(ii) 10 wt-% to 35 wt-%, based on the total weight of the powderous formulation, of an edible oil, and
(iii) at least 60 wt-%, based on the total weight of the powderous formulation, of sepiolite, and
(iv) 0 to 10 wt-%, based on the total weight of the powderous formulation, of water and/ or an additive.

4. The powderous formulation according to any of the preceding claims wherein the sepiolite has an average particle size D(v, 0.5) selected in the range of 100 to 1500µm.

5. The powderous formulation according to any of the preceding claims, wherein the sepiolite has an average particle size D(v, 0.5) selected in the range of 800 to 1200 µm, preferably in the range of 900 to 1100 µm.

6. The powderous formulation according to any of the preceding claims wherein the edible oil is selected from the group consisting of propyleneglycol, corn oil, rapeseed oil, sunflower oil, middle chain triglyceride (MCT) and glycerol as well as mixtures thereof.

7. The powderous formulation according to any of the preceding claims wherein the edible oil is propyleneglycol.

8. The powderous formulation according to any of the preceding claims of consisting of
(i) 4 to 8 wt-%, based on the total weight of the powderous formulation, of 3-nitrooxypropanol, and
(ii) 20 to 30 wt-%, based on the total weight of the powderous formulation, of propyleneglycol, and
(iii) at least 70 wt-%, based on the total weight of the powderous formulation, of sepiolite, and
(iv) 0 to 5 wt-%, based on the total weight of the powderous formulation, of water.

9. The powderous formulation according to any of the preceding claims, wherein the powderous form contains a coating.

10. The powderous formulation according to claim 9, wherein the coating is selected from the group consisting of glycerine monostearate, carnauba wax, candelilla wax, sugarcane wax, palmitic acid, stearic acid hydrogenated cottonseed oil, hydrogenated palm oil and hydrogenated rapeseed oil as well as mixtures thereof.

11. The powderous formulation according to any one of claim 1 to 10, wherein the retention of 3-nitrooxypropanol is at least 70%, preferably at least 80%, most preferably at least 90%.

12. Use of formulations according to anyone of claims 1 to 11 in feed products to enhance the retention of 3-nitrooxypropanol therein.

## Patentansprüche

1. Lagerstabile pulverförmige Formulierung, umfassend
(i) mindestens 0,1 Gewichtsprozent (Gew.-%), bezogen auf das Gesamtgewicht der pulverförmigen Formulierung, 3-Nitrooxypropanol,
(ii) 0 Gew.-% - 35 Gew.-%, bezogen auf das Gesamtgewicht der pulverförmigen Formulierung, eines essbaren Lösungsmittels und
(iii) mindestens 40 Gew.-%, bezogen auf das Gesamtgewicht der pulverförmigen Formulierung, Sepiolith.

2. Pulverförmige Formulierung nach Anspruch 1, ferner umfassend
(iv) 0 bis 10 Gew.-%, bezogen auf das Gesamtgewicht der pulverförmigen Formulierung, Wasser und/oder eines Additivs.

3. Pulverförmige Formulierung nach einem der vorhergehenden Ansprüche, bestehend aus
(i) 2 bis 15 Gew.-%, bezogen auf das Gesamtgewicht der pulverförmigen Formulierung, 3-Nitrooxypropanol und
(ii) 10 Gew.-% bis 35 Gew.-%, bezogen auf das Gesamtgewicht der pulverförmigen Formulierung, eines Speiseöls und
(iii) mindestens 60 Gew.-%, bezogen auf das Gesamtgewicht der pulverförmigen Formulierung, Sepiolith und
(iv) 0 bis 10 Gew.-%, bezogen auf das Gesamtgewicht der pulverförmigen Formulierung, Wasser und/oder eines Additivs.

4. Pulverförmige Formulierung nach einem der vorhergehenden Ansprüche, wobei der Sepiolith eine durchschnittliche Partikelgröße D(v,0,5) aufweist, die im Bereich von 100 bis 1500 µm ausgewählt ist.

5. Pulverförmige Formulierung nach einem der vorhergehenden Ansprüche, wobei der Sepiolith eine durchschnittliche Partikelgröße D(v,0,5) aufweist, die im Bereich von 800 bis 1200 µm, vorzugsweise im Bereich von 900 bis 1100 µm, ausgewählt ist.

6. Pulverförmige Formulierung nach einem der vorhergehenden Ansprüche, **dadurch gekennzeichnet, dass** das Speiseöl aus der Gruppe bestehend aus Propylenglykol, Maisöl, Rapsöl, Sonnenblumenöl, mittelkettigem Triglycerid (MCT) und Glycerin sowie Mischungen davon ausgewählt ist.

7. Pulverförmige Formulierung nach einem der vorhergehenden Ansprüche, wobei es sich bei dem Speiseöl um Propylenglykol handelt.

8. Pulverförmige Formulierung nach einem der vorhergehenden Ansprüche, bestehend aus
(i) 4 bis 8 Gew.-%, bezogen auf das Gesamtgewicht der pulverförmigen Formulierung, 3-Nitrooxypropanol und
(ii) 20 bis 30 Gew.-%, bezogen auf das Gesamtgewicht der pulverförmigen Formulierung, Propylenglykol und
(iii) mindestens 70 Gew.-%, bezogen auf das Gesamtgewicht der pulverförmigen Formulierung, Sepiolith und
(iv) 0 bis 5 Gew.-%, bezogen auf das Gesamtgewicht der pulverförmigen Formulierung, Wasser.

9. Pulverförmige Formulierung nach einem der vorhergehenden Ansprüche, wobei die pulverförmige Form einen Überzug enthält.

10. Pulverförmige Formulierung nach Anspruch 9, wobei der Überzug aus der Gruppe bestehend aus Glycerinmonostearat, Carnaubawachs, Candelillawachs, Zuckerrohrwachs, Palmitinsäure, Stearinsäure, hydriertem Baumwollsamenöl, hydriertem Palmöl und hydriertem Rapsöl sowie Mischungen davon ausgewählt ist.

11. Pulverförmige Formulierung nach einem der Ansprüche 1 bis 10, wobei die Retention von 3-Nitrooxypropanol mindestens 70 %, vorzugsweise mindestens 80 %, ganz besonders bevorzugt mindestens 90 %, beträgt.

12. Verwendung von Formulierungen nach einem der Ansprüche 1 bis 11 in Futterprodukten zur Verbesserung der Retention von 3-Nitrooxypropanol darin.

## Revendications

1. Formulation pulvérulente stable au stockage comprenant
(i) au moins 0,1 % en poids (% en poids), par rapport au poids total de la formulation pulvérulente, de 3-nitrooxypropanol,
(ii) 0 % en poids à 35 % en poids, par rapport au poids total de la formulation pulvérulente, d'un solvant comestible, et
(iii) au moins 40 % en poids, par rapport au poids total de la formulation pulvérulente, de sépiolite.

2. Formulation pulvérulente selon la revendication 1, comprenant en outre
(iv) 0 à 10 % en poids, par rapport au poids total de la formulation pulvérulente, d'eau et/ou d'un additif.

3. Formulation pulvérulente selon l'une quelconque des revendications précédentes, constituée de
(i) 2 à 15 % en poids, par rapport au poids total de la formulation pulvérulente, de 3-nitrooxypropanol, et
(ii) 10 % en poids à 35 % en poids, par rapport au poids total de la formulation pulvérulente, d'une huile comestible, et
(ii) au moins 60 % en poids, par rapport au poids total de la formulation pulvérulente, de sépiolite, et
(iv) 0 à 10 % en poids, par rapport au poids total de la formulation pulvérulente, d'eau et/ou d'un additif.

4. Formulation pulvérulente selon l'une quelconque des revendications précédentes, dans laquelle la sépiolite a une taille moyenne de particules D(v, 0,5) choisie dans la plage de 100 à 1 500 µm.

5. Formulation pulvérulente selon l'une quelconque des revendications précédentes, dans laquelle la sépiolite a une taille moyenne de particules D(v, 0,5) choisie dans la plage de 800 à 1 200 µm, de préférence dans la plage de 900 à 1 100 µm.

6. Formulation pulvérulente selon l'une quelconque des revendications précédentes, dans laquelle l'huile comestible est choisie dans le groupe constitué par le propylène glycol, l'huile de maïs, l'huile de colza, l'huile de tournesol, un triglycéride à chaîne moyenne (MCT) et le glycérol ainsi que des mélanges correspondants.

7. Formulation pulvérulente selon l'une quelconque des revendications précédentes, dans laquelle l'huile comestible est le propylèneglycol.

8. Formulation pulvérulente selon l'une quelconque des revendications précédentes, constituée de
(i) 4 à 8 % en poids, par rapport au poids total de la formulation pulvérulente, de 3-nitrooxypropanol, et
(ii) 20 à 30 % en poids, par rapport au poids total de la formulation pulvérulente, de propylène glycol, et
(ii) au moins 70 % en poids, par rapport au poids total de la formulation pulvérulente, de sépiolite, et
(iv) 0 à 5 % en poids, par rapport au poids total de la formulation pulvérulente, d'eau.

9. Formulation pulvérulente selon l'une quelconque des revendications précédentes, dans laquelle la forme pulvérulente contient un revêtement.

10. Formulation pulvérulente selon la revendication 9, dans laquelle le revêtement est choisi dans le groupe constitué par le monostéarate de glycérine, la cire de carnauba, la cire de candelilla, la cire de canne à sucre, l'acide palmitique, l'acide stéarique, l'huile de coton hydrogénée, l'huile de palme hydrogénée et l'huile de colza hydrogénée ainsi que des mélanges correspondants.

11. Formulation pulvérulente selon l'une quelconque des revendications 1 à 10, dans laquelle la rétention de 3-nitrooxypropanol est d'au moins 70 %, de préférence d'au moins 80 %, plus préférablement d'au moins 90 %.

12. Utilisation de formulations selon l'une quelconque des revendications 1 à 11 dans des produits alimentaires pour améliorer la rétention du 3-nitrooxypropanol dans ceux-ci.
